# EUROPEAN PATENT SPECIFICATION

(11) **EP 2 608 861 B1**
(45) Date of publication and mention of the grant of the patent: **28.11.2018**
(21) Application number: 11819477.8
(22) Date of filing: 16.08.2011
(51) Int. Cl.: B01D 15/00, C02F 9/00, C02F 1/28, C12N 1/12, C02F 1/463, C12N 1/02, C10L 5/44

(54) **CARBONACEOUS FINES BENEFICIATION USING MICRO-ALGAE AND RELATED PROCESSES**
AUFBEREITUNG KOHLENSTOFFHALTIGER FEINSTOFFE MITHILFE VON MIKROALGEN UND VERFAHREN DAFÜR
ENRICHISSEMENT DES PARTICULES FINES CARBONÉES EN UTILISANT DES MICRO-ALGUES ET PROCÉDÉS APPARENTÉS

(30) Priority: 23.08.2010 ZA 201005979
(43) Date of publication of application: 03.07.2013
(73) Proprietor: Nelson Mandela Metropolitan University, Summerstrand, Port Elizabeth 6031 (ZA)
(72) Inventor: ZEELIE, Bernard, 6045 Port Elizabeth Eastern Cape (ZA); DUGMORE, Gary, Morris, 6070 Port Elizabeth Eastern Cape (ZA)
(74) Representative: Browne, Robin Forsythe
(86) International application number: PCT/IB2011/001884
(87) International publication number: WO 2012/025806

(56) References cited:
- EP-B1- 0 930 273
- EP-B1- 0 930 273
- DE-A1-102006 016 715
- DE-A1-102006 016 715
- US-A- 5 676 836
- US-A- 5 676 836
- US-A1- 2010 167 339
- US-A1- 2010 167 339
- POELMAN E ET AL: "Potential of electrolytic flocculation for recovery of micro-algae", RESOURCES CONSERVATION AND RECYCLING, ELSEVIER SCIENCE PUBLISHER, AMSTERDAM, NL, vol. 19, no. 1, 1 January 1997 (1997-01-01), pages 1-10, XP004015353, ISSN: 0921-3449, DOI: 10.1016/S0921-3449(96)01156-1

## Description

### FIELD OF THE INVENTION

This invention relates broadly to the beneficiation of carbonaceous fines, especially those of coal and charcoal, using microalgae contained in an aqueous medium. The invention also relates to various processes related to the beneficiation of carbonaceous fines in this manner.

One such related process is the removal of microalgae from water with at least one objective being that of purifying the water whilst coincidentally beneficiating the carbonaceous fines.

Another related process is the production of agglomerates of the carbonaceous fines usually either in the form of briquettes being agglomerates moulded under elevated pressure and very often accompanied by curing at elevated temperature, or pellets that may be of a more random shape and size being the product of a generally conventional pelletization process.

Of course, a mixture of carbonaceous fines and micro-algae biomass may be fed directly to an appropriate process in which the mixture can be used, this especially being possible if the particle size of the carbonaceous fines, especially coal fines, is not too small.

In either event the admixture of carbonaceous fines and micro-algae may be utilized for heating, energy generation, mineral processing, generation of synthesis gas, and any other appropriate process.

It is to be understood that the terms carbonaceous fines as used in this specification is intended to include other suitable finely divided carbonaceous material such as finely divided coke and the like. Typically, any carbonaceous fines and that may be processed in accordance with the present invention would have a particle size of less than about 2 mm and would range down to extremely small particle sizes.

Also, the term agglomerate will be used to include briquettes, pellets and any other agglomerations of multiple particles.

### BACKGROUND TO THE INVENTION

Coal mining and processing produces enormous quantities of coal fines that cannot be used in most normal coal applications such as energy generation. If the coal fines are relatively coarse and free-flowing, they can often be recovered by front end loaders and other conventional earth-moving equipment, and are more than likely useful as fuel in fluidized bed boilers or specially-designed pulverized coal boilers.

Other potential uses of waste coal fines depend on more detailed quality characteristics, including size distribution; the nature and content of volatile matter; the sulfur content; the grindability; the ash content; other combustion characteristics; and moisture content.

When waste coal fines do not meet specific primary characteristics for utilization without further processing, they need to be formulated into a fuel product that does meet these primary characteristics. Various systems have been proposed for the reclamation and fuel product formulation of coal fines, mainly through one or other form of briquetting or pelletizing process as, for example, are described in US Patents 6,165,238, 6,530,966, and 6,626,966.

Some agglomeration processes require the addition of a binding agent, in most cases petroleum-derived binders, in order to give a stable and robust briquette or pellet. Other agglomeration processes do not utilize any additional binding agents and ensure the stability and robustness of briquettes or pellets by employing harsher processing conditions, mainly higher pressures, during briquetting or pelletizing and higher temperatures during curing.

Waste coal fines are typically deposited in ponds in the form of a slurry in water and the water subsequently evaporates to stabilize the deposit before it is covered. Such waste coal not only represents a huge economic loss, but the dumps also present a significant environmental problem as dry coal fines can ignite spontaneously and liberate huge quantities of carbon dioxide and other harmful gases into the atmosphere. Furthermore, leakages during wet periods, particularly of acidic water, can occur, and leaching of chemicals and minerals into the surrounding ground water is a real danger.

As a separate problem, many industrial activities, including commercial agricultural practices, mining practices, and municipal and industrial waste water treatment practices result in the contamination of surface fresh water sources such as dams and rivers. Such contaminated water systems often have substantial algal growth activity that results in what may be referred to as "greening" of such water systems.

Whilst algal growth in contaminated water systems can be beneficial as it removes nutrients and minerals from the water, it usually presents a substantial water quality problem since its removal from water systems is complicated.

Due to their small size, micro-algae are generally regarded as too difficult to remove by using conventional techniques such as sedimentation, filtration, and the like. If not removed from water systems, however, algae will most likely die off; return nutrients and minerals to the water as the dead algae decay; and thereby stimulate bacterial growth. Algae in water systems may also liberate quantities of malodorous chemicals into the water that makes it unsuitable for human and animal consumption.

DE 102006016715 discloses an agent for reducing or preventing the growth of green algae, preferably fibrous algae, in pools, rivers and tanks, and for suppressing rotten smell and odor emissions from water cultures and cropped plants, comprises lignite-based fiber material (brown coal xylite) and/or its granules.

### OBJECT OF THE INVENTION

It is an object of this invention to provide a method for the beneficiation of coal and charcoal fines using microalgae contained in an aqueous medium. It is another object of the invention to provide a method for the treatment of water containing microalgae. It is a further object of the invention to upgrade the quality of water containing microalgae. It is a still further object of the invention to provide a method by which coal and charcoal fines can be effectively agglomerated without the addition of petroleum-derived binders.

### SUMMARY OF THE INVENTION

According to an aspect of the invention there is provided a method for the harvesting and removing at least part of micro-algae from water, wherein the micro-algae are cultivated in commercial photo-bioreactor systems, the method being characterized in that it includes:
contacting carbonaceous fines with the water under conditions that permit adsorption of micro-algae onto particles of carbonaceous fines and in which the carbonaceous fines are loaded with micro-algae adsorbed thereon to an amount of from 1% to 25% micro-algae by weight of the carbonaceous fines;
separating the carbonaceous fines together with the adsorbed micro-algae thereon from the water; and
subjecting the carbonaceous fines together with the adsorbed micro-algae thereon to an agglomeration process for the production of agglomerates of the resultant admixture as part of a beneficiation of carbonaceous fines; and
wherein the micro-algae serve as a binder.

Further features of the invention provide for the carbonaceous fines to be coal fines; for the coal fines to be pre-treated, as may be required, to produce suitable size ranges of particles that may be aimed at facilitating adsorption, facilitating handling of the coal fines both before and after adsorption of the micro-algae and facilitating separation of the coal fines with adsorbed microalgae thereon from the water by a process appropriate to a selected particle size range of the coal fines; for the coal fines to be pre-treated for the removal of any unsuitable or valuable materials such as minerals therefrom; for contacting of the carbonaceous fines with microalgae containing water to take place either by forming a slurry of the carbonaceous fines in the water or by passing the water through a bed or column containing the carbonaceous fines; the carbonaceous fines may beloaded with microalgae adsorbed thereon to an amount of from 2% to 15% algae by weight of the carbonaceous fines, and most preferably from 5 to 10% by weight; and for the slurry to be subjected to electrochemical flocculation either during or after the micro-algae has become adsorbed onto the carbonaceous fines.

In one application of the invention the primary motivating factor may be the beneficiation of coal fines in which instance, if the coal fines have an appropriate size and constitution, the coal fines together with the microalgae adsorbed thereon may be used directly in a suitable application such as a fuel in fluidized bed boilers or specially-designed pulverized coal boilers.

In another application of the invention in which the primary motivating factor is the beneficiation of carbonaceous fines, the carbonaceous fines together with the adsorbed microalgae thereon may be subjected to an agglomeration process for the production of agglomerates typically in the form of briquettes or pellets of the resultant admixture. The agglomerates may be tailored for specific purposes and may include additional ingredients that can be added prior to or during the agglomeration process. They may also contain fines of different carbonaceous materials such as coal and charcoal in predetermined proportions.

In a still further application of the invention the primary motivating factor may be the harvesting of micro-algae cultivated in water in commercial photo- bioreactor systems specifically to reclaim and upgrade waste carbonaceous fines by agglomerating admixtures of carbonaceous fines and micro-algae biomass. In such an instance the water will typically be recirculated to the

photo-bioreactor system and, by so doing, the residual content of microalgae is preserved and indeed used to commence a new cycle of the microalgae growth process.

In yet another application of the invention the primary motivating factor may be the upgrading of polluted water systems that may be naturally occurring; a water system polluted by an increase in artificially produced nutrients that promote the growth of microalgae and that arise as a result of effluent from one or more factories; or a water system forming part of a commercial water treatment process.

Whatever the motivation, the invention makes available complementary technology for the upgrading of polluted water systems, while at the same time upgrading waste coal fines that are thus reclaimed.

In order that the invention may be more fully understood a more detailed discussion thereof and various examples and alternative arrangements will now be described with reference to the accompanying drawing.

### BRIEF DESCRIPTION OF THE DRAWING

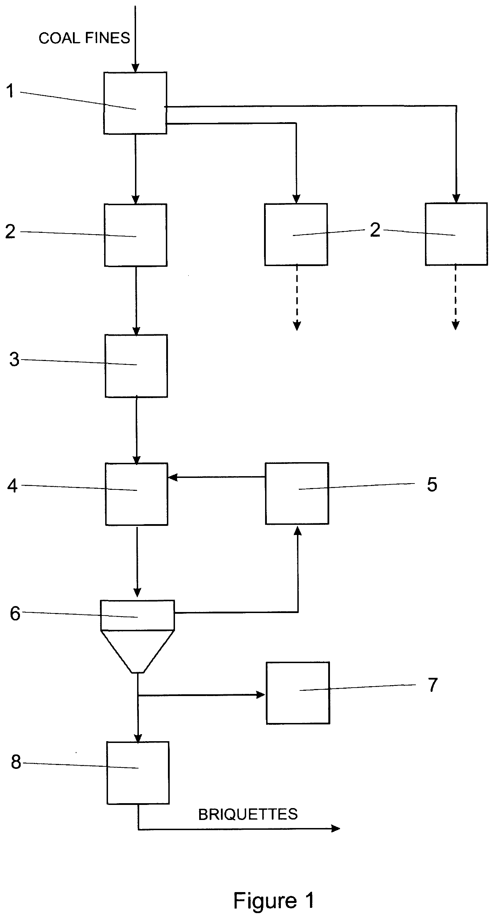

In the drawing Figure 1 is a flow diagram illustrating various possibilities of methods according to the invention.

### DETAILED DESCRIPTION WITH REFERENCE TO THE DRAWING

As indicated in the drawing, the method of this invention may, according to circumstances and requirements, include a sizing step [1] in which coal or other carbonaceous fines are pre-treated to produce at least one, and as may be appropriate, a plurality of suitable size ranges of particles, as indicated by numeral [2]. In the event that more than one size range is produced each size range can be treated differently according to the requirements of facilitating adsorption of micro algae, facilitating handling of the carbonaceous fines both before and after adsorption of the micro-algae and facilitating separation of the carbonaceous fines with adsorbed micro-algae thereon from the water, the method of separation typically being dictated largely by ultimate particle size. The manner in which the fines are sized is not critical and may, for example, be by screens, classifying cyclones, density-based processes such as spirals, hydrocyclones, and heavy-media cyclones.

Further, as may be required by the characteristics of the carbonaceous fines, they may be treated in a further pre-treatment step [3] for the removal of any unsuitable or valuable materials such as minerals. Unsuitable materials may be those that could adversely affect the performance of the agglomerates down the line, for example by creating a pollution problem when they are used to create thermal energy.

The main objective of pre-treatment is normally the removal of minerals from the fines and this may be achieved by conventional technologies. The pre-treatment may be selected so as to achieve specific processing and quality objectives. The size distribution of coal fines may be selected to allow rapid and effective adsorption of micro-algae whilst at the same time make separation of solids and liquid as easy and effective as possible.

Of course, the treatment of the coal fines to remove any unsuitable or valuable materials can take place ahead of the sizing step.

The present invention does not stipulate any restriction on the particle size of carbonaceous fines that can be used for the adsorption of micro-algae. It is, however, preferred that carbonaceous fines be sized so as to provide a reasonably equal size distribution.

The carbonaceous fines, after any pre-treatment, are contacted in a contact step [4] with water containing microalgae by a process appropriate to the particle size range of the coal fines. Contacting may typically be done either by forming a slurry of the coal fines in the algae containing water, or by passing the water through a bed or column containing the coal fines. In either event, contacting is carried out in a manner aimed at loading the carbonaceous fines with microalgae adsorbed thereon in a preferred amount of from 5 to 10% by weight.

The actual amount of micro-algae to be adsorbed will, in any event, depend on the eventual use of the carbonaceous fines-micro-algae mixture, as well as any method to be used for agglomerating the mixture. Thus, for low pressure, low temperature briquetting, it is advantageous to have higher amounts of micro-algae compared to an instance in which briquetting is done under higher briquetting pressures and curing temperatures.

To achieve desired micro-algae loadings, the amount of micro-algae in the source water is first determined on a dry mass per volume basis (e.g. grams/litre, grams/cubic meter, etc.) and from this the amount of carbonaceous fines and micro-algae containing water to be mixed is determined.

The water containing the microalgae may, as indicated above, be a naturally occurring or polluted water but will most typically be derived from commercial photo-bioreactor systems, as indicated by numeral [5], in which instance the contact constitutes harvesting of micro-algae cultivated in the water specifically for the purpose. In such an instance the water will generally be re-circulated to the photo-bioreactor system and, by so doing, any residual content of microalgae is preserved and indeed used to commence a new cycle of the microalgae growth process.

As may be required, any slurry would then be subjected to a separations step [6] the nature of which will depend largely on the size of the particles of carbonaceous fines. Thus, when relatively coarse coal fines are used, recovery from water may usually be achieved by sedimentation of the solids. When finer coal fines or other carbonaceous fines are used that do not settle easily, other techniques such as froth flotation may be used to recover solids from the slurry.

After separation, the carbonaceous fines together with the microalgae adsorbed thereon may be used according to requirements. If appropriate, they may be used directly in a suitable application such as a fuel in fluidized bed boilers or specially-designed pulverized coal boilers indicated by numeral [7].

Alternatively, and more commonly, the carbonaceous fines together with the adsorbed microalgae thereon will be subjected to an agglomeration step [8] in which briquettes or pellets of the coal fines are formed. The agglomerates may be tailored for specific purposes and may include additional ingredients that can be added prior to or during the agglomeration process.

Briquetting or pelletizing may be achieved in any suitable manner. Curing of the resulting briquettes or pellets may also be done using any known or other technology. Temperatures for curing may range from about 50° C to about 150° C. The exact temperature and time for curing is determined, at least to some extent, by the desired water content of the final agglomerate, and this in turn depends upon the use or application of the final briquettes or pellets. Thus, for gasification purposes, briquettes or pellets may have higher amounts of water in comparison to briquettes or pellets to be used for heating or energy generation purposes.

### EXAMPLES

### Example 1: Determination of the micro-algae loading capability of coal fines.

A mixture of microalgae was cultivated in a 2000L tubular photo-bioreactor and the micro-algae content contained in the growth medium determined as follows. Six centrifuge tubes of a Hitachi HIMAG centrifuge were preweighed (Table 1) and 5 ml of algae containing growth medium was introduced into each tube using a 5 mL pipette. The algae containing solutions were centrifuged for 10 minutes at 4000rpm and the supernatant solutions were decanted. The wet algae were dried (without washing) for 2 hours at 110 deg C; and the tubes cooled and re-weighed to allow calculation of the dry weight loading of algae (Table 1).

To 5 liters of the micro-algae containing water was added sufficient coal fines slurry (untreated raw slurry obtained from a Witbank mine, RSA and containing 124.8g solids per Kg of slurry) to give micro-algae loadings of 2.0, 5.0, 10.0 and 15.0 wt% (Table 2). The micro-algae coal fines mixture was mixed rapidly for approximately 20 seconds and then filtered through a filter cloth (200 g/m²) and the appearance of the first green residue that indicated free micro-algae in the slurry was noted. Only the mixture aimed at giving a final 15 wt% micro-algae showed free micro-algae in the mixture.

This example illustrates that (a) micro-algae are adsorbed by coal fines rapidly, and (b) coal fines can absorb micro-algae up to about 15 wt% based on the mass of coal fines used.

**Table 1: Microalgae loading in growth medium**

| **Tube (g)** | **Tube + Algae (g)** | **Algae (g)** | **Algae/L (g/L)** |
|---|---|---|---|
| 5.6883 | 5.6936 | 0.0053 | 1.06 |
| 5.5946 | 5.5996 | 0.005 | 1 |
| 5.6264 | 5.6314 | 0.005 | 1 |
| 5.4389 | 5.444 | 0.0051 | 1.02 |
| 5.5146 | 5.5201 | 0.0055 | 1.1 |
| 5.49 | 5.4956 | 0.0056 | 1.12 |
| **Average** | | | **1.05** |

**Table 2: Adsorbtion level determination**

| Mass microalgae (g) | Mass coal solids (g) | Volume coal slurry (L) | Residual algae (Yes/No)* |
|---|---|---|---|
| 5 | 257.25 | 2.06 | No |
| 5 | 99.75 | 0.80 | No |
| 5 | 47.25 | 0.38 | No |
| 5 | 29.75 | 0.24 | Yes |

| | | | |
|---|---|---|---|
| *Indicated by green colour on filter cloth/residual colour in water | | | |

### Example 2: Determination of the coal fines binding properties of micro-algae

Excess water was decanted from the micro-algae and coal fines slurry of example 1 and approximately 10 grams of the wet coal fines/micro-algae mixture was poured into a petri dish. This mixture was dried for 4 hours at 110°C and allowed to cool to room temperature. The hardness of the resultant cake was then tested by pressing the flat end of a stainless steel spatula into the cake. This was compared to a coal fines cake that contained no micro-algae.

The results show that the cake containing no micro-algae had little (if any) coherence of the particles in the cake; for coal fines having 2 and 5 wt% micro-algae, the cakes showed substantial increases in adherence, but still resulted in free dust formation when pressure was applied. At loadings of 10 and 15 wt% micro-algae, the cakes were hard enough to prevent the end of the spatula penetrating to the bottom of the cake. Shattering of these cakes did not result in the formation of dust-like particles, but rather large chunks of cake.

### Example 3: Effect of micro-algae on the settling rates of coal slurry

To 100 mL of coal slurry containing 12.48g of solids/L of slurry there were added various amounts of wet micro-algae biomass to give micro-algae loadings on coal of approximately 0, 5, 10 and 13% (Table 3). The volume of growth medium containing 1.05 g/L micro-algae (Example 1) were calculated and measured into four centrifuge tubes of a Hermle Z383 centrifuge. The algae were centrifuged for 10 minutes at 4000 rpm, the supernatant solution decanted and the coal slurry (100 mL) added. The algae and coal slurry were mixed thoroughly to ensure complete mixing of the micro-algae into the coal slurry.

The mixtures so derived were transferred into 100 mL measuring cylinders, and the rate of settling of the solids from the mixtures followed photographically using a Nikon P7000 digital camera using the interval time shooting mode, and using a 10 minute interval between pictures. The details of the mixtures are summarized in Table 3.

**Table 3: Settling rate of coal slurry**

| | | | |
|---|---|---|---|
| Mass coal solids (g) | 12.48 | | |
| g/L microalgae | 1.05 | | |

| Volumes of algae solution (L) | g coal solids used | Combined mass (g) | % Loading |
|---|---|---|---|
| 0.7 | 0.735 | 13.215 | 5.56 |
| 1.4 | 1.47 | 13.95 | 10.54 |
| 1.86 | 1.953 | 14.433 | 13.53 |

From a set of three pictures it was quite clear that the adsorbtion of micro-algae onto the surface of the coal slurry retards the settling rate of the coal fines. This observation is consistent with proposals (e.g. E. Poelman, N. De Pauw, and B. Jeurissen, Resources, Conservation and Recycling, 19 (1997), 1, 10) that micro-algae carry a natural negative charge which keeps the micro-algae from adhering to each other thereby stabilizing the micro-algae suspensions.

### Example 4: Electrochemical flocculation of coal fines and microalgae mixtures

The use of electrical current to cause the flocculation of microalgae is known in the art (e.g. E. Poelman, N. De Pauw, and B. Jeurissen, Resources, Conservation and Recycling, 19 (1997), 1, 10 and references therein) and since it was shown in Example 3 that microalgae also stabilize coal fines slurries, it was proposed that an electrical current can be used to flocculate slurries of coal fines containing microalgae. The electrochemical flocculation of a coal slurry containing 15% (m/m) micro-algae (prepared as described in Example 3) was compared to a coal slurry without any micro-algae.

An electrical current (10 V, 1.2 A) was passed through the two solutions using aluminium anodes and cathodes for about 10 minutes. No flocculation of the coal fines without microalgae could be observed at any time during the electrolysis, while the mixture containing coal and microalgae showed appreciable flocculation after only 10 minutes. The flocculation may therefore be considered to be rapid and the flocculated mixtures could be separated easily by decanting or filtering. The degree of settling of the coal fines/algae mixture was not as extensive as in the instance of the coal fines alone (after prolonged periods of standing- see Example 3) as the electrochemically generated flocs appeared to be substantially spongy.

It is believed that if the contact between the carbonaceous fines and the micro-algae is carried out in the presence of an electric current, higher loadings may well be achieved. This could prove to be advantageous especially in gasification and metallurgical processes.

### Example 5: Pelletizing coal fines/microalgae mixtures

Coal fines/micro-algae mixtures were prepared by mixing wet algae paste (obtained by centrifuging the microalgae from a volume of growth medium with known algae content) and coal slurry (500 mL containing 124.8 g/L solids). The mixture was stirred thoroughly and centrifuged at 4000 rpm for 5 minutes using a Hermle Z383 centrifuge. Water was decanted and the wet coal algae paste dried by pressing between sheets of filter paper.

The wet cake was divided into two approximately equal portions. The one portion was allowed to dry at room temperature for two days before pelletizing (Method A). The wet portion was pelletized immediately (Method B).

Pellets (approximately 1 g each) were prepared by compressing the wet slurry in a mould at a pressure of 144.8 bar (2100 pounds per square inch) using a hydraulic press. After removal from the press, pellets were dried at 160 deg C for 2 hours and cooled to room temperature. Pellets so prepared were subjected to two tests, namely a drop tests where pellets were dropped from a height of 1 meter onto a concrete floor and determining the % mass loss; and a water resistance test by submerging the pellets in water for 2 hours and observing their ability to retain their shape.

Table 4 below summarises the results obtained for the drop tests.

**Table 4: Mass loss observed in drop testing of pellets**

| Approximate % Micro-algae | Method A | | | Method B | | |
|---|---|---|---|---|---|---|
| | Pellet mass (g) | Mass after drop (g) | % Mass loss | Pellet mass (g) | Mass after drop (g) | % Mass loss |
| 0 | 1.0513 | 0.866 | 17.6% | 1.2198 | 1.0275 | 15.8% |
| 0 | 1.1034 | 0.9134 | 17.2% | 1.1873 | 1.0001 | 15.8% |
| 0 | 1.0087 | 0.8267 | 18.0% | 1.2096 | 1.0143 | 16.1% |
| 3.0 | | | | 1.0287 | 0.9042 | 12.1% |
| 5.0 | | | | 1.0743 | 1.0622 | 1.1% |
| 7.0 | 1.6795 | 1.6711 | 0.5% | 1.0858 | 1.0843 | 0.1% |
| 7.0 | 1.6294 | 1.6187 | 0.7% | 1.1278 | 1.1253 | 0.2% |
| 7.0 | 2.1947 | 2.1915 | 0.1% | 1.2689 | 1.2669 | 0.2% |
| 10.0 | | | | 1.0902 | 1.0866 | 0.3% |
| 15.0 | | | | 1.048 | 1.0446 | 0.3% |

The results of the weather resistance test showed that both algae free pellets, and algae-containing pellets prepared according to method A disintegrate after about 5 minutes after being submerged under water. Pellets containing algae prepared according to method B, and containing 5% (m/m) or more micro-algae, however, retain their shape even after 2 hours submerged in water. It was concluded that a minimum of 10% of micro-algae would render the pellets weather resistant form a practical viewpoint.

### Example 6: Pelletizing charcoal fines/microalgae mixtures

Charcoal/microalgae mixtures were prepared by mixing wet algae paste (obtained by centrifuging the microalgae from a volume of growth medium with known algae content - 3.0g microalgae on a dry weight basis) and charcoal (27.0 g - particle size < 500 µm). The mixture was mixed thoroughly by grinding together. Pellets (approximately 1 g each) were prepared by according to Method B of Example 5 and subjected to a drop test where pellets were dropped from a height of 1 meter onto a concrete floor and the % mass loss determined. Table 5 summarises the results obtained for the drop test of pellets containing no microalgae and pellets containing 10% microalgae.

**Table 5: Mass loss observed in drop testing of pellets**

| % Micro-algae | Pellet Mass (g) | Pellet mass after dropping (g) | % Mass loss |
|---|---|---|---|
| 0 | 1.2480 | 1.0498 | 15.9% |
| 0 | 1.5372 | 1.2629 | 17.8% |
| 0 | 1.4280 | 1.2237 | 14.3% |
| 0 | 1.3782 | 1.1446 | 17.0% |
| 10.0 | 1.0618 | 1.0551 | 0.63% |
| 10.0 | 1.0289 | 1.0255 | 0.33% |
| 10.0 | 1.1181 | 1.1152 | 0.26% |
| 10.0 | 1.0265 | 1.0233 | 0.31% |

Charcoal can therefore be successfully pelletized using microalgae as a binder with the pellets showing considerable handling strength, as indicated by the drop test results. Several pellets were ignited by briefly exposing them to a flame until the edge of the pellet started to glow. The burning process proceeded smoothly throughout the pellet which burnt without giving off any "chemical" smell. The ash remaining upon completion of the burning process was pure white and contained no remaining solid particles. The use of micro-algae as a binder appears to be a greatly improved way to bind charcoal for such purposes as barbequing, heating, etc since the pellets contain no extraneous chemical (as binders) and have a totally natural smell.

It is envisaged that mixtures of charcoal and coal fines with micro-algae may well provide a fuel that has advantageous burning properties, in particular, clean burning properties.

It has thus been found that micro-algae are strongly and rapidly adsorbed onto the surface of coal and charcoal fines and, this being so, a highly effective method of removal of micro-algae biomass from water in many different water systems is made available. Furthermore, the adsorbed micro-algae on the surface of the coal fines act as an effective binding agent so that coal fines may be easily and effectively agglomerated especially in the form of briquettes or pellets.

In appropriate circumstances, problematic micro-algae in fresh water supplies for human or animal usage may be removed effectively by adsorption onto the surface of carbonaceous fines. The quality of fresh water systems containing high levels of nutrients and minerals may be improved by removing micro-algae growing in such water systems on a continuous basis, thereby effectively removing the problematic nutrients and minerals.

It is to be noted that the energy value of coal fines is enhanced in the resultant agglomerates due to the significantly higher energy value of the micro-algae biomass as compared to the energy value of the coal. This results from the substantially higher hydrogen to carbon ratios of the micro-algae biomass of about 2.1 to 1 as compared to that of coal of about 0.7 to 1. In the instance of the production of synthesis gas the improved hydrogen to carbon ratios of micro-algae biomass and coal admixtures can result in significantly improved synthesis gas yields.

Whatever the motivation, the invention makes available complementary technology for the upgrading of polluted water systems, while at the same time upgrading waste carbonaceous and especially coal fines that are thus reclaimed.

Numerous different permutations and combinations of processes fall within the scope of the invention and the discussion above is not intended to be limiting in any way.

## Claims

1. A method for the harvesting and removing at least part of micro-algae from water, wherein the micro-algae are cultivated in commercial photo-bioreactor systems, the method being **characterized in that** it includes:
contacting (4) carbonaceous fines with the water under conditions that permit adsorption of micro-algae onto particles of carbonaceous fines and in which the carbonaceous fines are loaded with micro-algae adsorbed thereon to an amount of from 1% to 25% micro-algae by weight of the carbonaceous fines;
separating (6) the carbonaceous fines together with the adsorbed micro-algae thereon from the water; and
subjecting the carbonaceous fines together with the adsorbed micro-algae thereon to an agglomeration process (8) for the production of agglomerates of the resultant admixture as part of a beneficiation of carbonaceous fines; and
wherein the micro-algae serve as a binder.

2. A method as claimed in claim 1 in which the amount of micro-algae in the water is selected such that the micro-algae are adsorbed onto particles of fines in a quantity that is sufficient to produce agglomerates without petroleum-derived binders.

3. A method as claimed in either one of claims 1 or 2 in which agglomeration (8) involves briquetting or pelletization.

4. A method as claimed in any one of the preceding claims in which the carbonaceous fines are waste coal fines.

5. A method as claimed in any one of the preceding claims, comprising a sizing step (1) in which the carbonaceous fines are pre-treated to produce size ranges of particles (2).

6. A method as claimed in any one of the preceding claims in which contacting (4) of the carbonaceous fines with microalgae containing water takes place by forming a slurry of the carbonaceous fines in water.

7. A method as claimed in claim 6 in which the slurry is subjected to electrochemical flocculation either during or after the micro-algae has become adsorbed onto the carbonaceous fines.

8. A method as claimed in any one of the preceding claims in which the carbonaceous fines are loaded with microalgae adsorbed thereon to an amount of from 2% to 15% by weight.

9. A method as claimed in claim 8 in which the carbonaceous fines are loaded with microalgae adsorbed thereon to an amount of from 5 to 10% by weight.

10. A method as claimed in any one of the preceding claims, in which additional ingredients are added to the carbonaceous fines with the adsorbed micro-algae thereon prior to or during the agglomeration process (8).

11. The agglomerate as obtained by a method as claimed in any of claims 1 to 10, wherein the micro-algae serve as a binder of the carbonaceous fines in the agglomerates produced by the method.

## Patentansprüche

1. Verfahren zum Sammeln und Entfernen von zumindest einem Teil von Mikroalgen aus Wasser, wobei die Mikroalgen in kommerziellen Photobioreaktor-Systemen kultiviert werden,
wobei das Verfahren **dadurch gekennzeichnet ist,**
**dass** es Folgendes umfasst:
- Inkontaktbringen (4) von kohlenstoffhaltigen Feinstoffen mit dem Wasser unter Bedingungen, die die Adsorption von Mikroalgen auf Teilchen von kohlenstoffhaltigen Feinstoffen ermöglichen und bei denen die kohlenstoffhaltigen Feinstoffe mit darauf adsorbierten Mikroalgen in einer Menge von 1 % bis 25 % Mikroalgen, bezogen auf das Gewicht der kohlenstoffhaltigen Feinstoffe, beladen werden;
- Trennen (6) der kohlenstoffhaltige Feinstoffe zusammen mit den darauf adsorbierten Mikroalgen von dem Wasser; und
- Unterwerfen der kohlenstoffhaltigen Feinstoffe, zusammen mit den darauf adsorbierten Mikroalgen, einem Agglomerationsprozess (8) zur Herstellung von Agglomeraten des sich ergebenden Gemisches als Teil einer Aufbereitung von kohlenstoffhaltigen Feinstoffen; und
wobei die Mikroalgen als ein Binder dienen.

2. Verfahren gemäß Anspruch 1,
bei dem die Menge an Mikroalgen in dem Wasser so ausgewählt wird, dass die Mikroalgen auf Teilchen von Feinstoffen in einer Menge adsorbiert werden, die ausreicht, um Agglomerate ohne von Mineralöl abgeleitete Binder zu produzieren.

3. Verfahren gemäß einem der Ansprüche 1 oder 2,
bei dem die Agglomeration (8) ein Brikettieren oder Pelletisieren beinhaltet.

4. Verfahren gemäß einem der vorhergehenden Ansprüche ,
bei dem die kohlenstoffhaltigen Feinstoffe Abfallkohle-Feinstoffe sind.

5. Verfahren gemäß einem der vorhergehenden Ansprüche,
das einen Klassierschritt (1) umfasst, in dem die kohlenstoffhaltigen Feinstoffe vorbehandelt werden, um Größenbereiche von Teilchen (2) herzustellen.

6. Verfahren gemäß einem der vorhergehenden Ansprüche ,
bei dem das Inkontaktbringen (4) der kohlenstoffhaltigen Feinstoffe mit Mikroalgen enthaltendem Wasser durch das Bilden einer Aufschlämmung der kohlenstoffhaltigen Feinstoffe in Wasser erfolgt.

7. Verfahren gemäß Anspruch 6,
bei dem die Aufschlämmung entweder während oder nachdem die Mikroalgen auf den kohlenstoffhaltigen Feinstoffen adsorbiert worden sind, einer elektrochemischen Flockung unterworfen wird.

8. Verfahren gemäß einem der vorhergehenden Ansprüche,
bei dem die kohlenstoffhaltigen Feinstoffe mit darauf adsorbierten Mikroalgen in einer Menge von 2 bis 15 Gew.-% beladen werden.

9. Verfahren gemäß Anspruch 8,
bei dem die kohlenstoffhaltigen Feinstoffe mit darauf adsorbierten Mikroalgen in einer Menge von 5 bis 10 Gew.-% beladen werden.

10. Verfahren gemäß einem der vorhergehenden Ansprüche,
bei dem zusätzliche Bestandteile vor oder während des Agglomerationsprozesses (8) zu den kohlenstoffhaltigen Feinstoffen mit den darauf adsorbierten Mikroalgen zugegeben werden.

11. Agglomerat, erhalten durch ein Verfahren gemäß einem der Ansprüche 1 bis 10,
wobei die Mikroalgen als ein Binder der kohlenstoffhaltigen Feinstoffe in den durch das Verfahren hergestellten Agglomeraten dienen.

## Revendications

1. Procédé de récolte et d'enlèvement d'au moins une partie de micro-algues dans de l'eau, selon lequel les micro-algues sont cultivées dans des systèmes de photo-bioréacteur commerciaux, le procédé étant **caractérisé en ce qu'**il comporte :
la mise au contact (4) de fines carbonées avec de l'eau dans des conditions permettant l'adsorption de micro-algues sur les particules des fines carbonées, et selon lequel les fines carbonées sont chargées en micro-algues qui y sont adsorbées dans une teneur de 1 % à 25 % en poids de micro-algues par rapport au poids des fines carbonées ;
la séparation (6) des fines carbonées portant les micro-algues adsorbées de l'eau ; et
la soumission des fines carbonées portant les micro-algues adsorbées à un processus d'agglomération (8) pour la production d'agglomérés du mélange résultant en tant que partie d'une revalorisation des fines carbonées ; et
selon lequel les micro-algues servent de liant.

2. Procédé selon la revendication 1, selon lequel la teneur en micro-algues dans l'eau est choisie de telle sorte que les micro-algues sont adsorbées sur les particules de fines dans une teneur suffisante pour produire des agglomérés sans liants dérivés du pétrole.

3. Procédé selon l'une quelconque des revendications 1 ou 2, selon lequel l'agglomération (8) implique la mise sous forme de briquettes ou de pellets.

4. Procédé selon l'une quelconque des revendications précédentes, selon lequel les fines carbonées sont des fines de rebut de charbon.

5. Procédé selon l'une quelconque des revendications précédentes, comprenant une étape de tamisage (1) selon laquelle les fines carbonées sont pré-traitées afin de produire des gammes (2) de tailles de particules.

6. Procédé selon l'une quelconque des revendications précédentes, selon lequel la mise au contact (4) des fines carbonées avec des micro-algues contenant de l'eau s'opère par la génération d'une boue de fines carbonées dans de l'eau.

7. Procédé selon la revendication 6, selon lequel la boue est soumise à une floculation électrochimique, soit pendant, soit après, l'adsorption des micro-algues sur les fines carbonées.

8. Procédé selon l'une quelconque des revendications précédentes, selon lequel les fines carbonées sont chargées en micro-algues adsorbées dans une teneur de 2 % à 15 % en poids.

9. Procédé selon la revendication 8, selon lequel les fines carbonées sont chargées en micro-algues adsorbées dans une teneur de 5 % à 10 % en poids.

10. Procédé selon l'une quelconque des revendications précédentes, selon lequel des ingrédients supplémentaires sont ajoutés aux fines carbonées portant les micro-algues adsorbées, avant ou pendant le processus d'agglomération (8).

11. L'aggloméré tel qu'obtenu selon le procédé selon l'une quelconque des revendications 1 à 10, selon lequel les micro-algues servent de liant pour les fines carbonées dans les agglomérés produits par le procédé.
